Europäisches Patentamt

(19) European Patent Office

Office européen des brevets

(11) Publication number: **0 050 922**
**B1**

(12) **EUROPEAN PATENT SPECIFICATION**

(45) Date of publication of patent specification: **16.01.85**

(21) Application number: **81304533.3**

(22) Date of filing: **30.09.81**

(51) Int. Cl.⁴: **G 02 B 23/08**

(54) **Observation system for inspecting small diameter holes.**

(30) Priority: **27.10.80 JP 149292/80**
**08.11.80 JP 157217/80**
**26.11.80 JP 165262/80**
**03.06.81 JP 84479/81**

(43) Date of publication of application:
**05.05.82 Bulletin 82/18**

(45) Publication of the grant of the patent:
**16.01.85 Bulletin 85/03**

(84) Designated Contracting States:
**AT BE CH DE FR GB IT LI LU NL SE**

(56) References cited:
**CH-A- 110 148**
**DE-A-1 566 067**
**DE-A-2 135 981**
**DE-A-2 152 773**
**DE-A-2 911 478**
**DE-B-1 269 287**

(73) Proprietor: **SUMITOMO ELECTRIC INDUSTRIES LIMITED**
**No. 15, Kitahama 5-chome Higashi-ku**
**Osaka-shi Osaka-fu (JP)**

(72) Inventor: **Yoshida, Kenichi**
**1-3 Shimaya 1-chome**
**Konohana-ku Osaka (JP)**
Inventor: **Ono, Kimizo**
**1-3 Shimaya 1-chome**
**Konohana-ku Osaka (JP)**
Inventor: **Nishiwaki, Yoshikazu**
**1-3 Shimaya 1-chome**
**Konohana-ku Osaka (JP)**
Inventor: **Tsuno, Koichi**
**1-3 Shimaya 1-chome**
**Konohana-ku Osaka (JP)**
Inventor: **Iwai, Toru**
**1-3 Shimaya 1-chome**
**Konohana-ku Osaka (JP)**
Inventor: **Nishikawa, Mitsuru**
**1-3 Shimaya 1-chome**
**Konohana-ku Osaka (JP)**

(74) Representative: **Bayliss, Geoffrey Cyril et al**
**BOULT, WADE & TENNANT 27 Furnival Street**
**London EC4A 1PQ (GB)**

Courier Press, Leamington Spa, England.

## Description

The present invention concerns an observation system comprising a plural number of optical fibers bundled in alignment which is capable of focusing a picture image of small diameter holes located in front thereof at the forward end of said plural fiber bundle for observation of the picture image magnified by a magnifying lens which is transmitted through said optical fiber bundle at the back end thereof.

Observation system for inspecting small diameter holes is generally of the type wherein a plural number of optical fibers each of which constitutes a picture element is bundled in alignment so that a picture image of an object is focused by means of an image forming lens at the forward end of the optical fiber bundle, and transmitted through and enlarged by the magnifying lens of an eyepiece or the like at the back end thereof for inspection. Since the devices in such systems are extremely small in diameter and flexible in materials, they have been applied to a wide scope of the fields from industrial applications in various pipes such as heat conductive pipes used in heat exchangers at a chemical plant or a nuclear plant or a steel mill and medical applications to examine inside of a body such as cardiocameras or gastrocameras to civil usages such as security cameras or devices for monitoring dead angles in automobiles. The scope of this application is not limited to such uses but will extend further. One application in future will be to examine atmosphere filled with particles or volatile gases. Unlike conventional TV monitoring systems, the present system is completely free of the possibility to trigger electric sparks which might cause an explosion and endanger workers.

However, the conventional observation devices were extremely inconvenient when used to inspect the entire inner circumferential surface of a narrow pipe as, for one thing, it was necessary to employ an observation system having a bent tip which can be pivotally rotated, and for another, it was very difficult from outside to accurately locate the spot of observation and to orient the device toward the spot. It would be more convenient if the device is made capable of observing the frontal and lateral directions as it is fed into a pipe. The conventional devices were defective in this respect as well because they could not switch the direction of the observation between the frontal and lateral and thus posed various problems in practice. DE—A—2911478, DE—A—2135981 and DE—A—1566067 exemplify such prior arrangements.

The present invention aims to provide an observation system for inspecting small diameter holes which overcomes the above mentioned defects of the conventional devices and which is capable of inspecting the entire inner circumferential surface of a pipe without twisting the whole system, of switching the direction of observation between the frontal and the lateral and of accurately locating the spot to be observed and orienting from outside the device in that direction.

More specifically, the present invention comprises a first catoptric system which is fixed in front of a focusing lens in a rotatable manner to guide the light from the side toward said lens for inspecting the inner circumferential surface of a pipe as the system advances therethrough and a second catoptric system which is provided in an axially reciprocating manner at one side of said first catoptric system to guide therethrough the light from the frontal direction toward the focusing lens via said first catoptric system to enable observation in the frontal direction.

The system can be freely oriented for accurate and thorough inspection by remote-control of these catoptric systems. As the observation system is further provided with such members as a scale of angle and a magnetic needle which can be viewed superposed on the picture image of the object, the spot to be observed can be accurately located.

Fig. 1 is a sectional schematic view to illustrate the structure of one embodiment of the observation system according to the present invention.

Fig. 2 is an exploded perspective view to show the forward end of the system.

Fig. 3 illustrates one example of a vision of lateral observation.

Fig. 4 is a perspective view to show an example of a magnetic needle.

Fig. 5 is an exploded schematic view to show the structure of another embodiment at its forward end.

Fig. 6 is a sectional view to show one example of a remote control means.

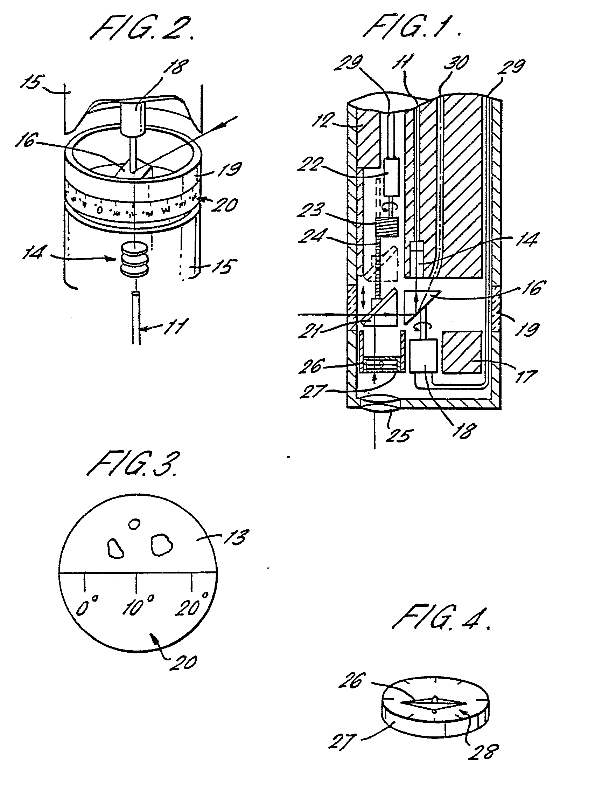

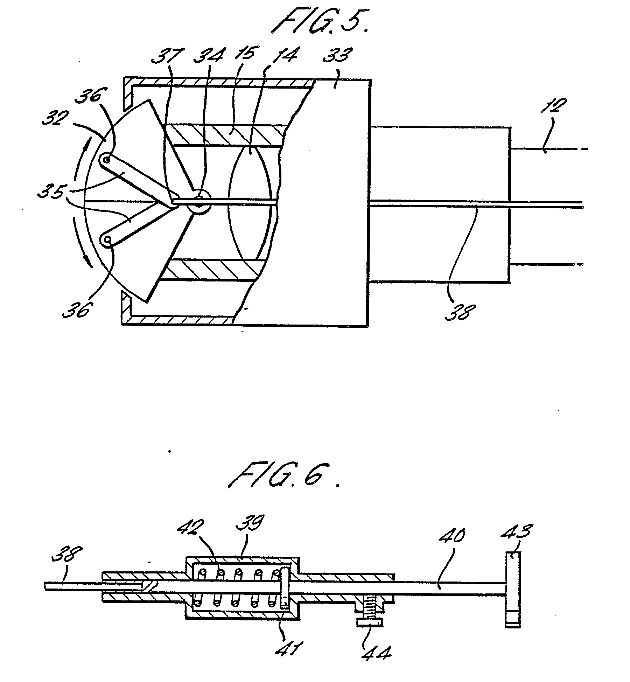

Referring to Figs. 1 through 4, one embodiment of the present invention will now be explained. An image transmission 11 comprising several to tens of thousands of optical fiber elements bundled in alignment (not shown) is protected by an elastic sheath 12. At the forward end of the sheath 12 is provided an image focusing lens 14 which focuses an image of an object 13 located frontal or lateral to the incident plane of the image fiber 11. A tubular image pickup adaptor 15 is also mounted on the sheath. The lens 14 in this embodiment is identical in structure with the graded type optical fibers in which the refractive index is variably distributed in the radial direction. In front of said lens 14 is fixed a total reflection prism 16 having the reflective plane inclined at an angle to the optical axis of the lens 14. The total reflection prism 16 reflects substantially at right angles the light from the inner circumferential surface of the object 13, guides for same toward the lens 14, and is connected for free rotation to a driving motor 18 for the prism which is supported by a holder 17 integrally with the image pickup adaptor 15. The portion of the image pickup adaptor 15 surrounding the prism 16 is made of transparent glass to define a window 19, which is provided with a scale of angle 20 (see Fig. 3). Thus, as one views the picture image of the object 13 at its inner circumferential surface through the window 19, one will be able to see the scale of angle 20 superposed on the picture image and be able to determine the direction of the observation as shown in Fig. 3.

Another total reflection prism 21 is positioned at one side of the prism 16 for guiding the light from the frontal direction toward the lens 14 via the prism 16 for frontal observation. Said prism 21 is supported by the sheath 12 in a reciprocating manner along the axial direction thereof. The prism 21 is integrally provided with transmission rack 24 which engages with driving worm 23 of a driving motor 22 for switching housed in said sheath 12, whereby the system can be selectively switched for frontal or lateral observation as the prism 21 is reciprocated along the axis of the sheath 12 by the driving motor 22. A transparent window 25 is formed in the image pickup adaptor 15 at its forward end located in front of the prism 21. A disk transparent case 27 which supports a magnetic needle 26 in a swinging manner by means of a pivot journal is fitted in the holder 17 between the window 25 and the prism 21. A scale 28 for the compass direction is printed on the surface of the cases 27 (as shown in Fig. 4). The case 27 is positioned normal to the light path leading to the prism 21. Thus, an image of the magnetic needle will also be superposed on a frontal picture image of the object 13 and this makes it possible to accurately orient the system for the frontal observation of the object 13 even if the sheath 12 is twisted.

Said motor 19 and the motor 22 are connected to a power source (not shown) at the back end of the sheath 12 via a wire 29 running in the sheath 12. A light guide 30 which is connected to a power source (not shown) at its base terminal in this embodiment passes through the sheath 12 and opens at the forward end thereof so that it opposes the prism 16. The object 13 is illuminated by the light projected from the light guide 30 and its reflective light is observed at the other end of the image transmission 11.

It is also possible to provide in an annular fashion the forward end of the light guide 30 around the focusing lens 14.

Although the worms 23, 24 and the motor 22 are used in this embodiment as the driving means for the prism 21 for lateral observation, a fluid pressure cylinder, a wire cable or a screw can also be employed. A reflective mirror can be used instead of the total reflection prisms 16 and 21. There will occur no inconveniences even if the optical axis of the lens 14 is not parallel to the incident light path for frontal observation.

For observing the inner circumferential surface of the object 13, the total reflection prism 16 alone is used while the prism 21 is moved backward to clear the view. For frontal observation, on the other hand, the prism 21 is positioned ahead of the prism 16 at its side and the prism 16 is rotated so that its reflective plane faces the prism 21.

Now, when the observation system is passed through an atmosphere laden with dusty particles, vapor or poisonous gas, such particles or vapor tend to adhere on the surface of the windows 25, 19 to deteriorate the quality of picture image or to chemically damage the windows. Another embodiment of the present invention aims to overcome this problem which will be described in more detail with reference to Figs. 5 and 6.

A cylindrical cover 33 is fixed axially slidable to the image pickup adaptor 15, said cover 33 housing a pair of open/close lids 32 (to be described later) and being adapted to be pulled backward by a remote control means using a wire cable and the like (not shown) at the time of observation. Said lids 32 are fixed to a pair of pins 34 which projects on the inner periphery of the cover 33 at an interval of 180° in a freely rotatable manner so that the lids can close the opening at the forward end of the cover 33. A pair of open/close links 35 is fixed at one end thereof to the open/close lids 32 by means of a pin 36 respectively and is connected to each other at the other end by means of a pin 37, to which is connected one end of a wire cable 38 which is arranged along the sheath 12. The other end of the wire cable 38 at the base of the sheath 12 is fixed integrally to a rod 40 which is attached to a grip holder 39 in a slidable manner. Inside the grip holder 39 is housed a compression coil spring 42 which abuts against a flange 41 formed on the rod 40 to constantly pressed the rod 40 toward the right as shown in Fig. 6. Therefore, if the top 43 of the rod 40 is pressed into the grip holder 39 against the compression of the coil spring 42, the wire cable 38 is made to move toward the left as shown in Fig. 5, which in turn makes the lids 32 rotate around the pin 34 via links 35 to open apart. In this embodiment, the lids 32 can be kept open by means of a screw 44 which is conveniently provided on the grip holder 39 without continuously pushing the top 43 of the rod 40. When the screw 44 is loosened, the rod 40 is pushed back toward the right by the force of the compression coil spring 42, thereby automatically closing the lids 32 via the wire cable 38 and the links 35.

## Claims

1. An observation system for inspecting small diameter holes comprising a bundle (11) of plural optical fiber elements for transmitting an image, a focusing lens (14) for focusing a picture image of an object on a forward end surface of said fiber elements in a tubular image pickup adaptor (15) which is fixed at the forward end of a sheath (12) for protecting said image fiber elements, a first catoptric system (16) provided in front of said focusing lens for observation from one direction having a reflective plane inclined with respect to the optical axis of said focusing lens, and driving means (18) provided inside said image pickup adaptor supporting said catoptric system in a rotatable manner, characterised in that a second catoptric system (21) for observation from a further direction is provided to one side of said first catoptric system, and a driving means (22, 23, 24) is provided supporting said second system (21) inside said image pickup adaptor to move said second catoptric system in a manner to switch the direction of observation.

2. The observation system for inspecting small diameter holes as claimed in claim 1 wherein an annular transparent window (19) is formed outside the image pickup adaptor (15) surrounding said catoptric systems (16, 21) and said window is provided with a scale of angle (20).

3. The observation system for inspecting small diameter holes as claimed in claim 1 or 2 wherein a magnetic needle (26) indicating compass direction is attached to said image pickup adaptor (15) located in front of said second catoptric system (21).

**Patentansprüche**

1. Beobachtungssystem zur Kontrolle von Löchern geringeren Durchmessers mit einem aus einer Vielzahl von optischen Faserelementen bestehenden Bündel (11) zum Senden eines Bildes, mit einer Fokussierlinse (14) zur Fokussierung eines Objektbildes auf die nach vorn liegende Stirnfläche der Faserelemente in einem am nach vorn liegendem Ende einer zum Schutz der Bildfaserelemente bestimmten Hülle befestigten rohrförmigen Bildaufnahmeadapter (15), mit einer ersten vor der Fokussierlinse (14) liegenden katoptrischen Einrichtung (16) zur Beobachtung aus der einen Richtung, wobei die Spiegelebene der Einrichtung (16) zur optischen Achse der Fokussierlinse (14) geneigt ist, ferner mit einer innerhalb des Bildaufnahmeadapters (15) liegenden, die Einrichtung (16) drehbar stützenden Antriebsvorrichtung (18), dadurch gekennzeichnet, dass eine zweite nach einer Seite der ersten (16) liegende, zur Beobachtung aus einer weiteren Richtung bestimmte katoptrische Einrichtung (21) vorgesehen ist, wobei eine diese zweite katoptrische Einrichtung (21) innerhalb des Bildaufnahmeadapters (15) stützende Antriebsvorrichtung (22, 23, 24) diese zweite katoptrische Einrichtung (21) derart verschiebt, dass die Beobachtungsrichtung umgeschaltet wird.

2. Beobachtungssystem zur Kontrolle von Löchern geringeren Durchmessers nach Anspruch 1, dadurch gekennzeichnet, dass ein ringförmiges durchscheinendes Fenster (19) ausserhalb des um die katoptrischen Einrichtungen (16, 21) herumliegenden Bildaufnahmeadapters (15) gebildet ist, wobei das Fenster (19) eine Winkelskala (20) aufweist.

3. Beobachtungssystem zur Kontrolle von Löchern geringeren Durchmessers nach einem der Ansprüche 1 und 2, dadurch gekennzeichnet, dass eine für Richtungsanzeige bestimmte Magnetnadel (26) mit dem vor der zweiten katoptrischen Einrichtung (21) liegenden Bildaufnahmeadapter (15) verbunden ist.

**Revendications**

1. Système d'observation pour l'inspection de trous de petit diamètre comprenant un faisceau (11) formé de plusieurs éléments à fibres optiques pour transmettre une image, une lentille de focalisation (14) pour la focalisation de l'image d'un objet sur une surface terminale avancée desdits éléments à fibres dans une monture tubulaire (15) de captation d'image qui est fixée à l'extrémité avant d'un manchon (12) pour la protection desdits éléments à fibres image, un premier système catoptrique (16) prévu devant ladite lentille de focalisation pour l'observation dans un sens ayant un plan réflecteur incliné par rapport à l'axe optique de ladite lentille de focalisation, et des moyens d'entraînement (18) prévus à l'intérieur de ladite monture de détection d'image soutenant ledit système catoptrique d'une manière rotative, caractérisé en ce qu'un deuxième système catoptrique (21) pour l'observation d'un autre sens est prévu sur un côté dudit premier système catoptrique, et en ce qu'un mécanique d'entraînement (22, 23, 24) est prévu soutenant ledit deuxième système (21) à l'intérieur de ladite monture de détection d'image pour déplacer ledit deuxième système catoptrique de manière à changer le sens d'observation.

2. Le système d'observation pour l'inspection de trous de petit diamètre tel que revendiqué à la revendication 1 caractérisé en ce qu'une fenêtre transparente annulaire (19) est formée à l'extérieur de la monture de détection d'image (15) entourant lesdits systèmes catoptriques (16, 21) et en ce que ladite fenêtre est dotée d'une échelle d'angles (20).

3. Le système d'observation pour l'inspection de trous de petit diamètre tel que revendiqué à la revendication 1 ou 2, caractérisé en ce qu'une aiguille magnétique (26) indiquant la direction de boussole est reliée à ladite monture de détection d'image (15) située devant ledit deuxième système catoptrique (21).

FIG. 2.

FIG. 1.

FIG. 3.

FIG. 4.

FIG.5.

FIG.6.